# FASCICULE DE BREVET EUROPEEN

(11) **EP 2 803 005 B1**
(45) Date de publication et mention de la délivrance du brevet: **06.04.2016**
(21) Numéro de dépôt: 13701856.0
(22) Date de dépôt: 09.01.2013
(51) Int. Cl.: G07F 17/00, G07F 11/62, A61B 10/00, B65G 1/137

(54) **DISPOSITIF ET PROCEDE DE STOCKAGE ET D'ORDONNANCEMENT DE BLOCS DE PRELEVEMENT BIOLOGIQUE**
VORRICHTUNG UND VERFAHREN ZUR AUFBEWAHRUNG UND ANORDNUNG VON KASSETTEN FÜR BIOLOGISCHE PROBEN
APPARATUS AND METHOD FOR STORAGE AND ARRANGEMENT OF BIOLOGICAL SPECIMEN CASSETTES

(30) Priorité: 10.01.2012 FR 1250244
(43) Date de publication de la demande: 19.11.2014
(73) Titulaire: Dreampath Diagnostics, 67000 Strasbourg (FR)
(72) Inventeur: WILHELM, Valérie, F-67400 Illkirch-Graffenstaden (FR)
(74) Mandataire: Rhein, Alain
(86) Numéro de dépôt international: PCT/FR2013/050052
(87) Numéro de publication internationale: WO 2013/104865

(56) Documents cités:
- EP-A1- 0 706 825
- WO-A1-2010/004331
- WO-A2-99/60982
- WO-A2-2005/054992
- US-A1- 2001 032 035
- US-A1- 2007 135 965

## Description

La présente invention entre dans le domaine médical de l'analyse de prélèvements tissulaires et cellulaires.

L'invention concerne plus particulièrement la conservation et le stockage de tels prélèvements.

Dans le cadre de la prise en charge médicale d'un patient ou dans le cadre de la recherche, des prélèvements tissulaires ou cellulaires peuvent être effectués en vue d'une analyse histologique et/ou moléculaire. A des fins de conservation, ces éléments sont déshydratés puis conservés inclus en parrafine dans un support, appelé communément « blocs de paraffine ». Après réalisation des coupes nécessaires à l'analyse histologique et/ou moléculaire, les résidus tissulaires et cellulaires inclus en paraffine sont conservés afin de pouvoir accéder ultérieurement à des analyses complémentaires (parfois des années plus tard).

De tels blocs de paraffine sont généralement constitués d'un boîtier en matériau plastique de forme standardisée, parallélépipédique rectangle, dont le fond est ajouré par des orifices traversants. De plus, une des parois latérales, généralement la paroi avant, reçoit des informations uniques d'identification de l'échantillon, telle une référence. Une telle paroi peut être prévue inclinée, afin de faciliter la lecture desdites informations.

Plusieurs exemples de tels boîtiers sont décrits au travers des documents US D448 487 S, US 4 421 246 ou GB 2 113 249. De manière générique, ces boîtiers sont appelés « cassettes ».

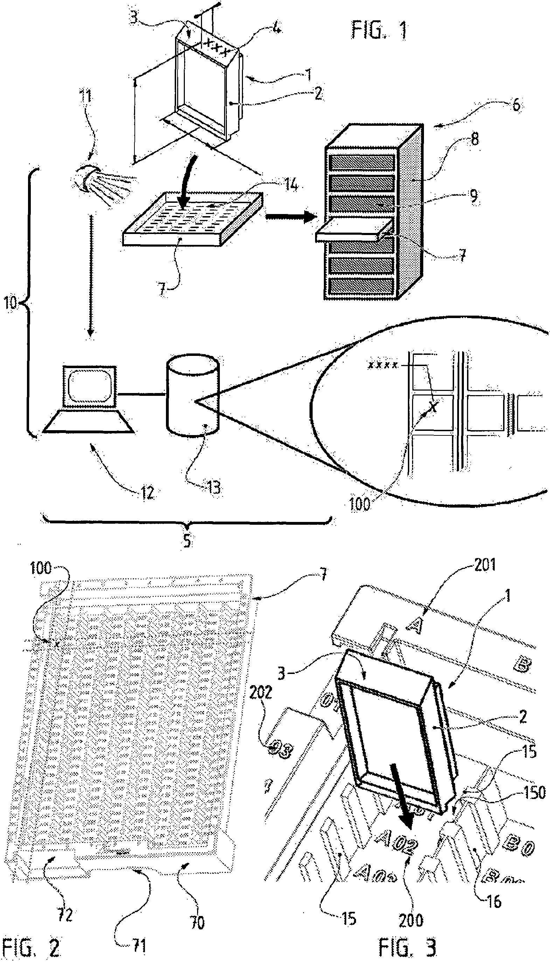

Un détail de la figure 1 montre une vue en perspective d'un exemple de boîtier ou cassette en position verticale, face supérieure tournée vers l'avant. Une telle cassette présente une hauteur de 41,8 millimètres (mm), pour 28,5 mm de large et 6,5 mm de hauteur ou d'épaisseur.

C'est donc au sein d'une telle cassette que le prélèvement biologique est déposé et refermé provisoire par un couvercle amovible qui est ensuite retiré au moment de l'inclusion en paraffine, la face supérieure laissant apparaître la paraffine et pouvant être débitée ultérieurement avec l'échantillon qu'elle enferme. La cassette et le prélèvement inclus sous paraffine constituent alors un bloc de paraffine.

Ces blocs et leur échantillon y conservé sont répertoriés et stockés par différents établissements, notamment les laboratoires d'analyse médicale. Légalement, leur conservation est obligatoire pour chaque individu sur une durée de dix années minimum pour une entreprise privée, allant jusqu'à plusieurs décennies pour un établissement public ou une idustrie pharmaceutique.

A l'heure actuelle, lesdits blocs sont rangés et manipulés manuellement, par des opérateurs n'ayant forcément reçu aucune formation spécifique, sans aucun contrôle. De ce fait, une telle gestion manuelle entraine des erreurs et une perte de temps considérable dans leur recherche, allant jusqu'à la perte préjudiciable de certains prélèvements.

De plus, dans ce contexte, les quantités de blocs existantes sont en constante évolution : d'environ 200 millions par an dans les années 1990, leur nombre a augmenté à près de 400 millions par an en 2010 et cette croissance est estimée à 750 millions par an vers 2030. Dès lors, les capacités de stockage et les moyens empiriques mis en oeuvres actuellement ne sont pas prévus pour supporter la gestion d'un stockage rationnel et sûr pour de telles quantités.

A l'heure actuelle, il n'existe aucune solution appropriée et dédiée au stockage et la gestion de tels blocs. Il convient toutefois de noter que d'autres systèmes de gestion de matériel médical existent, sans pour autant être adaptés.

Le document US 2007/135965 décrit un dispositif de stockage df'objets médicaux, offrant une gestion de la traçablité de leur usage. Un tel dispositif se présente sous la forme d'une armoire pourvue de plateaux ou tiroirs, divisés intérieurement en compartiments, par des parois saillantes verticalement. En particulier, ces compartiments présentent des dimensions adaptables, en déplaçant lesdites parois, pour modifier leur taille respective en fonction du format des objets qu'ils sont destinés à recevoir.

De plus, ce dispositif intègre des moyens informatiques de gestion de l'accès à chaque compartiment. En particulier, ces moyens permettent de lire un code présent sur chaque objet et d'en référencer les informations y relatives. En outre, ces moyens de gestion permettent de retrouver rapidement et aisément, à l'aide d'une interface tactile, offrant une visualisation graphique précise, le compartiment où se trouve l'objet recherché.

Une autre solution est décrite dans le document WO 99/60982 concernant un dispositif de boîtier à couvercle articulé, destiné à recevoir intérieurement des médicaments. En particulier, un tel boîtier peut être positionné au sein d'un tiroir, prévu à cet effet. Lors de leur rangement, plusieurs boîtiers sont positionnés côte à côte selon des colonnes et des rangées. De plus, une fiche saillante en face inférieure du fond de chaque boîtier est destinée à coopérer par clipsage au sein d'une fente ménagée en face supérieure au sein du fond dudit tiroir. Chaque boîtier comprend aussi intérieurement des moyens d'identification électronique qui, lors de leur emboîtement, au sein du tiroir, permettent d'étalibr une connexion et d'identifier avec exactitude la position de chaque boîtier au sein du tiroir.

Encore une autre solution est décrite dans le document US 2001/032035, concernant un plateau destiné à être placé au sein d'un tiroir. Ledit plateau comprend des parois intérieures amovibles, de manière à en diviser l'intérieur en plusieurs compartiments, dont les dimensions sont adaptables en fonction des objets qu'ils vont recevoir.

De tels systèmes ne sont donc clairement pas adaptés à la gestion d'articles tous identiques, de petites dimensions, comme les blocs de paraffine. En outre, les systèmes de l'état de la technique ne prévoit aucun positionnement particulier des objets, tous différents et de formes hétérogènes, qu'ils envisagent de stocker sans prendre en compte les particularités propres à chacun d'entre eux.

En outre, on notera que des technologies connues sont mises en oeuvre pour faciliter l'identification et la traçabilité d'objets dans le domaine médical. A titre d'exemple, la technologie RFID (pour « Radio Frequency IDentification ») peut être utilisée au travers de puces électroniques accolées sur de tels objets, assurant leur identification automatique au travers d'un lecteur sans fil, possédant un champ de détection à courte portée (« Near Field Communication »). Le lecteur étant connecté à un gestionnaire informatique distant, ce dernier permet d'effectuer un suivi des objets utilisés et d'en gérer leur approvisionnement. Un exemple d'une telle solution est envisagée au travers du document WO 2005/054992.

Une solution similaire consiste à équiper un support de tube de prélèvement, de type éprouvette, d'une mémoire informatique à l'intérieur de laquelle peuvent être stockées des informations relatives audit prélèvement. Il n'est alors plus nécessaire d'apposer les informations directement sur le tube de prélèvement. Un exemple d'une telle solution est décrite dans le document EP 0 706 825.

Toutefois, ces solutions offrent une technologie pour contenir des informations relatives à des objets, sans pour autant en assurer le classement et le rangement, en particulier pour de grandes quantités d'objets identiques.

La présente invention a pour but de pallier les inconvénients de l'état de la technique, en proposant un système d'ordonnancement informatisé et automatisé de blocs de prélèvement biologique. Un tel système se veut à même d'offrir une traçabilité et une gestion rationnelle de blocs, pour des quantités importantes, notamment réparties dans des lieux géographiques distincts.

Pour ce faire, un tel système prévoit tout d'abord des moyens de stockage de plusieurs blocs, par positionnement au sein d'au moins un compartiment de stockage horizontal, selon des colonnes et rangées, de manière à être disposées verticalement, face avant comportant les informations d'identification tournée vers le haut. De plus, l'invention ne fait intervenir aucun ordonnancement au niveau d'un tel stockage, les blocs étant positionnées sans ordre précis de rangement, lors du remplissage des colonnes et rangées.

En particulier, le caractère d'ordonnancement découle de ce stockage particulier au travers de moyens automatiques de lecture desdites informations présentes sur chaque blocs et de référencement informatisé de la position de ledit bloc au sein des moyens de stockage.

Ainsi, la présente invention concerne tout d'abord un dispositif de stockage et d'ordonnancement de bloc de prélèvement biologique, lesdits blocs se présentant sous la forme d'un boîtier équipé sur une face d'informations d'identification. Un tel dispositif se caractérise par le fait qu'il comprend, d'une part, des moyens de stockage constitués d'au moins un élément sous forme de tiroir s'étendant horizontalement de positionnement desdits blocs selon des colonnes et des rangées, verticalement ladite face tournée vers le haut au travers de moyens d'emboîtement ménagés sous forme de saillies ou redents, espacés régulièrement, ménageant ainsi des parties mâles et femelles au niveau du fond, au sein desquelles peuvent être enfichées lesdits blocs et, d'autre part, des moyens de référencement de la position de chaque bloc au sein desdits moyens de stockage, lesdits moyens de référencement comprenant des moyens de lecture des informations d'identification de chaque bloc et d'enregistrement informatisé de ladite position.

De plus, selon d'autres caractéristiques, lesdits redents peuvent être ménagés en saillie le long et de part et d'autre de parois intérieures verticales, orientées de manière à définir des colonnes.

Préférentiellement, lesdits moyens de stockage peuvent comprendre au moins une armoire de stockage de plusieurs éléments se présentant alors sous la forme de tiroirs.

Selon un mode de réalisation, lesdits moyens de référencement peuvent comprendre des moyens informatisés de recherche de ladite position d'un bloc à partir de ses informations d'identification.

En particulier, lesdits moyens de lecture peuvent comprendre un lecteur de type scanner lumineux de données encodées, notamment de type code à barres ou code matriciel.

L'invention concerne aussi un procédé de stockage et d'ordonnancement de bloc de prélèvement biologique, lesdits blocs se présentant sous la forme d'un boîtier équipé sur une face d'informations d'identification, dans lequel :
- on positionne, selon des colonnes et des rangées, verticalement ladite face vers le haut, au moins un bloc au sein d'un élément de stockage s'étendant horizontalement, de type tiroir, pourvu de moyens d'emboîtement ménagés sous forme de saillies ou redents, espacés régulièrement, ménageant ainsi des parties mâles et femelles au niveau du fond, constituant des espaces au sein desquels peuvent être enfichées lesdits bloc ; et
- on effectue un référencement de la position de ledit bloc au sein dudit élément de stockage par lecture desdites informations d'identification et l'enregistrement informatisé de leur position.

Dès lors, l'invention assure un rangement adapté et conformé spécifiquement au format desdits blocs, dimensionné en conséquence. Ce rangement dédié permet ainsi de stocker un grand nombre de blocs, tout en connaissant avec exactitude leur emplacement.

A ce titre, le positionnement des blocs se veut arbitraire, de sorte qu'un opérateur peut les placer sans se soucier d'un emplacement ou d'un ordre prédéterminé, diminuant le temps nécessaire à un classement qui est effectué automatiquement par identification de chaque bloc et de son emplacement.

Ainsi, l'invention permet de stocker rapidement et simplement, sans nécessité de formation particulière de l'opérateur, un grand nombre de blocs qui seront automatiquement classées et localisées au travers d'un outil informatique, assurant leur totale traçabilité.

D'autres caractéristiques et avantages de l'invention ressortiront de la description détaillée qui va suivre des modes de réalisation non limitatifs de l'invention, en référence aux figures annexées, dans lesquelles :
- la figure 1 représente schématiquement l'architecture du système de stockage et d'ordonnancement selon l'invention ;
- la figure 2 représente une vue en perspective d'un élément de stockage de type tiroir selon un mode de réalisation préférentiel ; et
- la figure 3 représente une vue schématisée d'un détail de la figure 2, faisant apparaître un bloc lors de son insertion verticalement, face avant tournée vers le haut, en une position dudit tiroir.

La présente invention concerne le stockage et l'ordonnancement de blocs 1 de prélèvement biologique.

On notera que de tels blocs 1 servent de support de conservation de prélèvement biologique, comme des tissus ou cellules. Elles se présentent sous la forme d'un boîtier 2, notamment parallélépipédique rectangle, équipé sur une face d'informations d'identification, notamment au niveau de la paroi extérieure de la face avant 3 dudit bloc 1. Cette face avant 3 est d'ailleurs souvent prévu inclinée, afin de faciliter la lecture desdites informations, lorsque le bloc 1 est repose au niveau de son fond.

Ces dernières permettent d'identifier de manière unique la personne à qui appartient ledit prélèvement, ainsi que d'autres données connexes, comme par exemple, de façon non exhaustive, la date et le type du prélèvement ou bien les analyses déjà réalisées sur un ou plusieurs échantillons de ce prélèvement.

On notera que ces informations peuvent être imprimées sur chaque bloc 1 sous forme de données encodées. Ces dernières peuvent être apposées au moment de leur fabrication et correspondent alors à un numéro unique pour chaque bloc 1.

A titre d'exemple, lesdites données peuvent se présenter sous la forme d'un encodage de type code à barres ou code matriciel 4, lisible par un scanner adapté, notamment lumineux.

De plus, ledit numéro unique peut être mis en correspondance, lors du placement du prélèvement au sein de son bloc 1, avec lesdites informations d'identification. Cette mise en correspondance peut être effectuée par affectation du numéro unique au sein d'un dossier médical informatisé propre à chaque personne, dans le domaine de la santé, mais aussi de la recherche.

Avantageusement, dans un premier temps, la présente invention concerne un dispositif 5 de stockage et d'ordonnancement de telles blocs 1. Ce dispositif 5 se veut à même d'être complètement dédié à cette gestion de blocs 1 tous identiques, facilitant leur rangement ainsi que leur classement, afin d'en retrouver rapidement et simplement l'emplacement de chacune d'entre elles, peu importe le nombre.

Pour ce faire, d'une part, ledit dispositif 5 comprend des moyens de stockage 6 constitués d'au moins un élément 7 de positionnement desdits blocs 1 selon des colonnes et des rangées.

Plus particulièrement, un tel élément 7 s'étend horizontalement et permet le positionnement desdits blocs 1 verticalement, ladite face 3 tournée vers le haut. Ainsi, il est possible de lire, visuellement ou bien automatiquement par un système adapté, les informations d'identification de chaque bloc 1 lorsqu'il est stocké au sein d'un élément 7 du dispositif 5.

Selon le mode de réalisation préférentiel, lesdits moyens de stockage 6 peuvent comprendre au moins une armoire 8 de stockage de plusieurs éléments 7 se présentant alors sous la forme de tiroirs. Ces derniers sont alors superposés et/ou juxtaposés. En particulier, plusieurs tiroirs peuvent être montés en coulissement au sein de logements 9 correspondants ménagés au sein de ladite armoire 8, selon des colonnes, des rangées ou des deux.

Selon l'exemple de réalisation représenté sur la figure 1, une telle armoire 8 peut se présenter sous la forme d'une unité verticale comprenant des logements 9 pour recevoir sept tiroirs. Une telle armoire peut toutefois être dimensionnée pour recevoir plus ou moins de tiroirs, selon plus d'une colonne.

En outre, chaque armoire 8 peut prévoir des moyens de fermeture hermétique, telle des portes, permettant d'assurer le maintien de l'intérieur de ladite armoire 8 sous une atmosphère particulière, notamment réfrigérée, dépressurisée ou bien sous un gaz spécifique. Cette particularité assure une meilleure conservation des prélèvements ainsi stockés.

On notera que chaque armoire 8 et chaque élément tiroir 7 peuvent recevoir des informations uniques d'identification, tel un numéro de série, permettant de les identifier de manière unique et d'être mis en correspondance les uns avec les autres, à savoir que plusieurs tiroirs 7 peuvent être associés à une armoire 8, notamment à ses logements 9 ou bien à un de ses logements 9 en particulier.

Dans ce dernier cas de figure, lesdits logements 9 peuvent comprendre de moyens de réception en coulissement de chaque tiroir 7, sous la forme de détrompeurs, possédant des moyens de réception complémentaire au niveau dudit tiroir 7, assurant qu'un tiroir 7 est bien positionné au sein du logement 9 qui lui est destiné.

De tels détrompeurs peuvent se présenter sous la forme d'une glissière, coulisse, rainure ou gorge particulière équipant ledit tiroir 7 au niveau de ses parois extérieures, en particulier ses parois latérales. Ces détrompeurs peuvent alors former alors une partie mâle ou femelle destinée à coopérer avec réciproquement une partie femelle ou mâle ménagées au sein de chaque logement 9.

Plus avant, selon le mode de réalisation préférentiel, comme représenté sur les figures, ledit dispositif 5 comprend des éléments 7 sous forme de tiroirs. Afin de permette le positionnement vertical des blocs 1, facilitant leur identification une fois en place, chaque tiroir 7 présente au niveau de son fond des moyens d'emboîtement 14. Ces derniers sont ménagés sous forme de saillies ou redents 15, espacés régulièrement, ménageant ainsi des parties mâles et femelles au niveau dudit fond, au sein desquelles peuvent être enfichées lesdits blocs 1. En somme, l'espace entre chaque saillie ou redent 15 constitue un logement ou une fente 150 de réception en emboîtement d'un bloc 1.

Selon le mode de réalisation visible sur la figure 3, lesdits redents 15 sont ménagés en saillie au niveau du fond, à savoir en partie inférieure du tiroir, mais le long et de part et d'autre de parois intérieures verticales 16. Ces dernières sont orientées parallèlement, à intervalles réguliers, de manière à définir des colonnes au sein dudit élément 7, à savoir ledit tiroir.

On notera que l'espacement entre les saillies ou rendents 15 est dimensionné de manière à autoriser l'emboîtement de chaque bloc 1, avec ou sans jeu, préférentiellement sans jeu. Dans ce dernier cas, la largeur de chaque espacement, à savoir la distance entre les surfaces de deux saillies ou redents 15 consécutifs, est quasiment égal à l'épaisseur d'un bloc 1, de un à plusieurs dixièmes de millimètres près. Ainsi, une fois emboîtée, chaque bloc 1 est maintenu au sein de son emplacement, même si le tiroir venait à se retourner.

Ainsi, l'espacement entre les saillies correspond sensiblement à l'épaisseur d'un bloc 1, de manière à assurer son emboîtement sans jeu ou avec un jeu minime, ledit bloc 1 étant alors emboîtée en force et maintenue, même en cas de renversement du tiroir 7.

Selon l'exemple de blocs de paraffine évoqué en partie introductive, la largeur d'une rangée peut être d'environ 29 millimètres (mm), de préférence 29,2 mm pour un bloc de 28,5 mm de large. L'espacement entre les saillies ou redents 15 peut alors être d'environ 6 mm, de préférence 6,6 mm, pour un bloc de 6,6 mm d'épaisseur. Enfin, la hauteur du tiroir peut être d'environ 50 à 60 mm, de préférence 53,5 mm, pour un bloc d'une longueur de 41,8 mm, tandis que la hauteur des parois médianes 16 peut être inférieure, notamment d'au moins 10 à 30 mm, laissant ainsi sortir le haut des blocs 1 permettant de facilité leur préhension.

En outre, l'épaisseur desdites saillies, ergots ou redents 15 permet la préhension et l'extraction, manuelle ou automatique, notamment robotisée, des blocs 1. Pour ce faire, selon un mode particulier de réalisation, lesdites saillies ou redents 15 peuvent s'étendre uniquement sur une partie de la hauteur de chaque paroi médiane intérieure 16. De préférence, la partie supérieure de chaque paroi 16 est dépourvue de redents 15, ces derniers ne s'étendant que depuis le fond sur une hauteur moindre que celle des parois latérales qui l'entourent, à savoir deux parois 16 ou bien les bords latéraux dudit élément 7.

De plus, selon un mode préférentiel de réalisation, chaque tiroir peut recevoir en face supérieure ouverte, un couvercle amovible, permettant de refermer le tiroir, notamment en vue de sa manutention. On notera que le jeu, entre la face inférieure dudit couvercle et les blocs 1 positionnés au sein du tiroir 7, est tel que lesdits blocs 1 ne peuvent pas changer de position. En somme, cet espace ne permet pas à un bloc 1 de sortir complètement de son logement, si le tiroir est retourné.

Selon un autre mode de réalisation, les faces supérieure et inférieure de chaque tiroir 7 peuvent recevoir réciproquement des moyens d'emboîtement et d'emboîtement complémentaires, conformés de manière à permettre leur superposition en maintien les uns au dessus des autres. En somme, les moyens d'emboîtement peuvent consister en des parties mâles venant s'insérer au sein de parties femelles constituées par lesdits moyens complémentaires, ou inversement. Ainsi, il est possible de gerber plusieurs tiroirs 7.

Dès lors, lorsque plusieurs tiroirs 7 sont empilés, seul le tiroir supérieur peut être refermé par ledit couvercle.

Selon le mode préférentiel de réalisation, chaque tiroir 7 peut comprendre un espace réservé 72 à des données assurant son identification de manière unique. Ces données peuvent être de tout type, notamment encodées de manière à être lue par des scanners lumineux, de type lecteur de code à barres ou analogue.

De plus, ledit espace réservé 72 peut être ménagé au niveau d'un des bords du tiroir 7, notamment situé au niveau de sa face avant 70, sous la forme d'un biseau sur lequel sont imprimées lesdits données d'identification. Ce biseau offre une surface inclinée dont l'orientation facilite la lecture desdites données, manuellement ou automatiquement.

Selon le mode de réalisation de la figure 2, ledit tiroir 7 peut comprendre en face avant 70 des moyens de préhension, sous forme d'une poignée 71, notamment ménagée au seins de la paroi par un espace réservé. Cette poignée 71 permet d'insérer et d'extraire le tiroir 7 de l'armoire 8, mais aussi de le transporter comme une valise, lorsqu'il est refermé par son couvercle.

D'autre part, ledit dispositif 5 comprend des moyens 10 de référencement de la position 100 de chaque bloc 1 au sein desdits moyens de stockage 6. De tels moyens de référencement 10 comprennent des moyens 11 de lecture des informations d'identification, en particulier des données encodées 4, de chaque bloc 1 et d'enregistrement informatisé de ladite position 100 de chaque bloc 1.

En somme, une fois que le bloc 1 est positionnée au sein du tiroir 7, les moyens de lecture 11 enregistre sa position 100 exacte, notamment à l'aide de coordonnées bidimensionnelles, à deux chiffres, selon les rangées et les colonnes correspondant réciproquement aux abscisses et aux ordonnées. Une colonne et une rangée ont été représentées schématiquement en pointillé sur la figure 2, où l'intersection constitue une position. De plus, les données dudit tiroir 7 peuvent être ajoutées, en guise de coordonnées supplémentaires, ledit tiroir 7 étant alors destiné à être inséré au sein d'une armoire 8, dont l'identification peut aussi être ajoutée. On obtient alors un système de référencement de la position de chaque bloc 1 allant de deux à quatre dimensions.

Dès lors, il est possible pour chaque bloc 1, de connaître sa position 100 exacte en fonction des coordonnées liées aux différentes données d'identification des différentes parties du dispositif 5 selon l'invention. On notera que cette position 100 peut ensuite être enregistrée dans une base de données numériques 13, équipée d'un gestionnaire, afin de faciliter son accès ultérieur.

A l'inverse, une fois cette position 100 enregistrée, le dispositif 5 permet de la retrouver. Pour ce faire, lesdits moyens de référencement 10 comprennent des moyens 12 informatisés de recherche de ladite position 100 d'une bloc 1 à partir de ses informations d'identification. Ces moyens de recherche 12 peuvent se présenter sous la forme d'un terminal informatique, équipé de moyens de saisie, manuelle ou automatique, notamment par lesdits moyens de lecture 11. En somme, un opérateur peut entrer un code, un numéro d'identification ou bien scanner une étiquette sur laquelle est imprimée les données d'un bloc 1, et les moyens de recherche 12 interroge ladite base de données 13 pour extraire la position 100 et localiser ledit bloc 1 au sein du dispositif 5.

Ensuite, l'opérateur n'a plus qu'à ouvrir le tiroir correspondant et localiser ladite position. A ce titre, chaque tiroir 7 peut comprendre au niveau de des faces supérieures de ses différentes parois (telles le fond, les bords périphériques ou les chants supérieurs des parois verticales) des données numériques 200 identifiant chaque rangée et chaque colonne. En particulier, selon le mode de réalisation préférentiel visible sur les figures 2 et 3, des lettres 201 peuvent identifier les colonnes, tandis que des nombres 202 peuvent identifier les rangées. Ainsi, la position la plus haute et la plus à gauche, située dans le coin supérieur gauche, possède la position A01, la position juste à droite B01, tandis que la position juste en dessous A02.

De plus, sur la face supérieure du fond du tiroir 7, à savoir intérieurement, au niveau de chaque logement 150, peut apparaître ladite position 200 (A01, B01, A02...), ménagée en saillie ou en creux. En outre, ce numéro de position peut être située entre les saillies ou rendents 15, permettant à un utilisateur de la visualiser plus facilement, même quand un bloc 1 y est inséré.

En outre, dans le cas d'un tiroir 7 en un matériau plastique, ces numéros et lettres sont directement moulés lors de la fabrication, empêchant toute modification ultérieure.

Selon une caractéristique additionnelle, l'invention permet de vérifier la présence ou non d'un bloc 1 au sein de chaque position du tiroir. A partir de cette vérification des positions, des comparaisons peuvent être envisagées avec les positions déjà connues, afin de vérifier le mauvais placement, le déplacement ou le retrait d'une bloc 1. En cas de différence constatée, une alerte peut être émise afin d'en informer un opérateur.

On notera que, selon un mode particulier de réalisation, des clichés photographiques numériques de chaque bloc peuvent être réalisés automatiquement lors de leur positionnement. Ces clichés permettent de faciliter le repérage ultérieur du bloc, notamment en raison de sa couleur, mais assurera aussi une saisie manuelle d'une identification de bloc 1 suite à une mauvaise lecture, mais aussi la saisie de son identification si cette dernière n'était pas équipée d'une identification sous forme de données encodées 4. Ce cliché servira aussi de preuve sur la présence physique de ces blocs 1 à des fins de traçabilité, et/ou pour des stockages extérieurs aux locaux du laboratoire, de l'hôpital ou des centres de Recherche académiques ou privés.

On notera que, selon un mode préférentiel de réalisation, lesdits moyens de lecture 11 comprennent un lecteur de type scanner lumineux de données encodées 4, notamment de type code à barres ou code matriciel, à une ou deux dimensions. Un tel lecteur peut être utilisé et manipulé manuellement, ou bien de façon automatique.

Dans ce dernier cas, l'invention peut prévoir d'effectuer une lecture automatique de toutes les données 4 de chaque bloc 1 stocké dans un tiroir, dès que ce dernier est placé au niveau des moyens de lecture 11.

A ce titre, ces derniers peuvent être intégrés à chaque armoire et une lecture de chaque tiroir est effectuée lorsque ce dernier est remis en place, après une extraction préalable, ou refermé s'il avait été ouvert.

Ainsi, la lecture peut permettre de détecter tout changement de place, ajout ou retrait d'un bloc, par rapport au classement préalable, déjà connu et stocké, au travers d'une simple comparaison.

De plus, lesdits moyens de recherche 12 permettent d'assurer le suivi de la manutention d'un bloc 1. En somme, une traçabilité est possible, de façon automatique et transparente pour l'utilisateur, permettant de savoir si elle est stockée dans le dispositif 5 ou si elle en a été retirée, par quelle personne et ainsi suivre son parcours.

En outre, lors de son retour au sein du dispositif 5, le positionnement du bloc 1 peut s'effectuer à une place différente, choisie de façon arbitraire par l'opérateur, et le système met à jour ladite position 100 de façon automatique, par une étape de lecture. Il est alors possible de positionner les blocs 1 sans se soucier d'un ordre précis, mais tout en conservant avec certitude l'emplacement exacte en vue de les retrouver. En somme, un opérateur peut positionner un bloc 1 au sein de tout emplacement libre, accélérant le rangement dans le cas de plusieurs blocs.

Dès lors, il est possible d'envisager une surveillance globale de l'intégralité du système, des dispositifs 5 implantés et des positions de toutes les blocs 1 existants, permettant de les localiser géographiquement.

L'invention concerne aussi un procédé de stockage et d'ordonnancement de blocs 1 de prélèvement biologique, lesdits blocs 1 se présentant sous la forme d'un boîtier 2 équipé sur une face 3 d'informations d'identification, dans lequel :
- on positionne, selon des colonnes et des rangées, verticalement ladite face 3 vers le haut, au moins un bloc 1 au sein d'un élément 7 de stockage s'étendant horizontalement, de type tiroir, pourvu de moyens d'emboîtement ménagés sous forme de saillies ou redents 15, espacés régulièrement, ménageant ainsi des parties mâles et femelles au niveau du fond, constituant des espaces au sein desquels peuvent être enfichées lesdits blocs 1 ; et
- on effectue un référencement de la position 100 de ledit bloc 1 au sein dudit élément 7 de stockage par lecture desdites informations d'identification et l'enregistrement informatisé de leur position 100.

Comme évoqué précédemment, le positionnement de chaque bloc 1 peut être effectué de façon arbitraire au sein des espaces des moyens d'emboîtement.

Ainsi, la présente invention permet de ranger simplement et rapidement des blocs 1, de les conserver dans cet état ordonné, tout en assurant une traçabilité des prélèvement qu'elles contiennent.

En outre, l'invention permet de s'adapter à des solutions de stockage existantes au sein des laboratoires, hôpitaux, centres de recherche privés ou académique, mais peut aussi être transposée à un centre distinct pour une externalisation totale et rationalisée du stockage et du référencement des blocs.

## Revendications

1. Dispositif (5) de stockage et d'ordonnancement de blocs (1) de prélèvement biologique, lesdits blocs (1) se présentant sous la forme d'un boîtier (2) équipé sur une face (3) d'informations d'identification imprimées sur chaque bloc (1) sous forme de données encodées, notamment de type code à barres ou code matriciel, ledit dispositif (5) comprenant, d'une part, des moyens (6) de stockage constitués d'au moins un élément (7) sous forme de tiroir s'étendant horizontalement de positionnement desdits blocs (1) selon des colonnes et des rangées, verticalement ladite face (3) tournée vers le haut au travers de moyens d'emboîtement ménagés sous forme de saillies ou redents (15), espacés régulièrement, ménageant ainsi des parties mâles et femelles au niveau du fond, au sein desquelles peuvent être enfichées lesdits blocs (1) et, d'autre part, des moyens (10) de référencement de la position de chaque bloc (1) au sein desdits moyens de stockage (6), **caractérisé par le fait que** lesdits moyens de référencement (10) comprennent des moyens (11) de lecture automatique des informations d'identification, en particulier desdites données encodées, de chaque bloc (1) et d'enregistrement informatisé et de mise à jour automatique de ladite position bidimensionnelle au sein dudit tiroir, lesdits moyens de lecture (11) comprenant un lecteur de type scanner lumineux des données encodées.

2. Dispositif (5) selon la revendication 1, **caractérisé par le fait que** lesdits redents (15) sont ménagés en saillie le long et de part et d'autre de parois intérieures verticales (16), orientées de manière à définir des colonnes.

3. Dispositif (5) selon l'une quelconque des revendications précédentes, **caractérisé par le fait que** lesdits moyens de stockage (6) comprennent au moins une armoire (8) de stockage de plusieurs éléments (7) se présentant alors sous la forme de tiroirs.

4. Dispositif (5) selon l'une quelconque des revendications précédentes, **caractérisé par** le fais que lesdits moyens de référencement (10) comprennent des moyens informatisés de recherche de ladite position d'un bloc (1) à partir de ses informations d'identification.

5. Procédé de stockage et d'ordonnancement de blocs (1) de prélèvement biologique, lesdits blocs (1) se présentant sous la forme d'un boîtier (2) équipé sur une face (3) d'informations d'identification imprimées sur chaque bloc (1) sous forme de données encodées, notamment de type code à barres ou code matriciel, dans lequel :
- on positionne verticalement ladite face (3) vers le haut, selon des colonnes et des rangées, au moins un bloc (1) au sein d'un élément (7) de stockage s'étendant horizontalement, de type tiroir, pourvu de moyens d'emboîtement ménagés sous forme de saillies ou redents (15), espacés régulièrement, ménageant ainsi des parties mâles et femelles au niveau du fond, constituant des espaces au sein desquels peuvent être enfichées lesdits blocs (1) ; et
- on effectue un référencement de la position bidimensionnelle dudit bloc (1) au sein dudit élément de stockage (7) par lecture automatique desdites informations d'identification, en particulier desdites données encodées, au moyen d'un lecteur de type scanner lumineux, et l'enregistrement informatisé et la mise à jour automatique de leur position.

## Patentansprüche

1. Vorrichtung (5) zur Aufbewahrung und Anordnung von Kassetten (1) für biologische Proben, wobei die Kassetten (1) die Form eines Kastens (2) haben, der auf einer Seite (3) mit Identifizierungsinformationen ausgestattet ist, die auf jede Kassette (1) in Form von codierten Daten, vor allem von Strich- oder Matrixcodes, gedruckt sind, wobei die Vorrichtung (5) zum einem Aufbewahrungsmittel (6), die von mindestens einem Element (7) in Schubkastenform gebildet sind, das sich horizontal zur Positionierung der Kassetten (1) gemäß Kolonnen und Reihen erstreckt, wobei die Seite (3) anhand von Rastmitteln, die in Form von gleichmäßig beabstandeten Vorsprüngen oder Stufen (15) ausgebildet sind und dadurch männliche und weibliche Abschnitt im Bereich des Bodens ausbilden, in welche die Kassetten (1) einschiebbar sind, vertikal nach oben zeigt, und zum anderen Referenzierungsmittel (10) der Position jeder Kassette (1) innerhalb der Aufbewahrungsmittel (6) umfasst, **dadurch gekennzeichnet, dass** die Referenzierungsmittel (10) automatische Lesemittel (11) der Identifizierunginformationen, insbesondere der codierten Daten, jeder Kassette (1) und informatisierte Registrierungs- und automatische Aktualisierungsmittel der zweidimensionalen Position innerhalb des Schubkastens umfassen, wobei die Lesemittel (11) ein Lesegerät vom Typ Lichtscanner der codierten Daten umfassen.

2. Vorrichtung (5) nach Anspruch 1, **dadurch gekennzeichnet, dass** die Stufen (15) hervorspringend entlang und beiderseits von vertikalen Innenwänden (16) ausgebildet sind, die derart ausgerichtet sind, dass sie Kolonnen bilden.

3. Vorrichtung (5) nach einem der vorangehenden Ansprüche, **dadurch gekennzeichnet, dass** die Aufbewahrungsmittel (6) mindesten einen Aufbewahrungsschrank (8) von mehreren Elementen (7) umfasst, die dann die Form von Schubkästen haben.

4. Vorrichtung (5) nach einem der vorangehenden Ansprüche, **dadurch gekennzeichnet, dass** die Referenzierungsmittel (10) informatisierte Suchmittel der Position einer Kassette (1) mit Hilfe ihrer Identifizierungsinformationen umfassen.

5. Verfahren für die Aufbewahrung und Anordnung von Kassetten (1) für biologische Proben, wobei die Kassetten (1) die Form eines Kastens (2) haben, der auf einer Seite (3) mit Identifizierungsinformationen ausgestattet ist, die auf jede Kassette (1) in Form von codierten Daten, vor allem von Strich- oder Matrixcodes, gedruckt sind, wobei:
- die Seite (3) mindestens einer Kassette (1) gemäß den Kolonnen und Reihen innerhalb eines sich horizontal erstreckenden Aufbewahrungselements (7) vom Typ Schubkasten, ausgestattet mit Rastmitteln, die in Form von gleichmäßig beabstandeten Vorsprüngen oder Stufen (15) ausgebildet sind und dadurch männliche und weibliche Abschnitt im Bereich des Bodens ausbilden, die Bereiche ausbilden, in welche die Kassetten (1) einschiebbar sind, vertikal nach oben positioniert wird, und
- eine Referenzierung der zweidimensionalen Position der Kassette (1) innerhalb des Aufbewahrungselements (7) durch automatisches Lesen der Identifizierungsinformationen, insbesondere der codierten Daten, mittels eines Lesegeräts vom Typ Lichtscanner, und die informatisierte Registrierung und die automatische Aktualisierung ihrer Position durchgeführt wird.

## Claims

1. Device (5) for storing and ordering biological sampling blocks (1), said blocks (1) being in the form of a housing (2) provided on one surface (3) with identification information printed on each block (1) in the form of encoded data, namely such as a bar code or matrix code, said device (5) comprising, on the one hand, means (6) for storing formed of at least one element (7) in the form of a horizontally-extending drawer for positioning said blocks (1) vertically in columns and rows, said surface (3) facing upward, through nesting means provided in the form of regularly spaced projections or recesses (15), thus forming male and female parts at the level of the bottom, in which said blocks (1) can be inserted and, on the other hand, means (10) for referencing the position of each block (1) within said storing means (6), wherein said referencing means (10) comprise means (11) for automatically reading the identification information, in particular of said encoded data, of each block (1) and for the computerized recording and automatic updating of said two-dimensional position within said drawer, said reading means (11) comprising a reader such as a light scanner for encoded data.

2. Device (5) according to claim 1, wherein said recesses (15) are formed projecting along and on both sides of vertical inner walls (16) oriented so as to define columns.

3. Device (5) according to any of the preceding claims, wherein said storage means (6) comprise at least one storage cupboard (8) of several elements (7) that are then in the form of drawers.

4. Device (5) according to any of the preceding claims, wherein said referencing means (10) comprise computerized means for searching said position of a block (1) based on its identification information.

5. Method for storing and ordering biological sampling blocks (1), said blocks (1) being in the form of a housing (2) provided on one surface (3) with identification information printed on each block (1) in the form of encoded data, namely such as a bar code or matrix code, wherein:
- at least one block (1) is positioned vertically, in columns and rows, said face (3) facing upwards, within a horizontally extending storage element (7), such as a drawer, provided with nesting means arranged in the form of regularly spaced projections or recesses (15), thereby providing male and female parts at the level of the bottom, forming spaces in which said blocks (1) can be inserted; and
- a referencing of the two-dimensional position of said block (1) in said storage element (7) is carried out by automatic reading said identification information, in particular of said encoded data, by means of a reader such as a light scanner and computerized recording and automatic updating of their position.
